# EUROPÄISCHE PATENTSCHRIFT

(11) **EP 3 615 931 B1**
(45) Veröffentlichungstag und Bekanntmachung des Hinweises auf die Patenterteilung: **18.08.2021**
(21) Anmeldenummer: 18721328.5
(22) Anmeldetag: 25.04.2018
(51) Int. Cl.: G01N 33/49, B01L 3/00, B01D 21/26, A61B 5/15

(54) **TRENNKÖRPER**
SEPARATING BODY
CORPS DE SÉPARATION

(30) Priorität: 26.04.2017 DE 102017108933
(43) Veröffentlichungstag der Anmeldung: 04.03.2020
(73) Patentinhaber: Sarstedt Aktiengesellschaft & Co. KG, 51588 Nümbrecht (DE)
(72) Erfinder: WEINSTOCK, Mark, 57612 Helmenzen (DE); WEGENER, Christian, 51588 Nümbrecht (DE); KARRENBERG, Ulrich, 51709 Marienheide (DE)
(74) Vertreter: Grosse, Wolf-Dietrich Rüdiger
(86) Internationale Anmeldenummer: PCT/EP2018/060597
(87) Internationale Veröffentlichungsnummer: WO 2018/197562

(56) Entgegenhaltungen:
- EP-A2- 0 017 127
- EP-A2- 1 106 252
- WO-A1-2010/132783
- WO-A2-2014/120678
- US-A- 3 887 464
- US-A1- 2002 132 367

## Beschreibung

Die Erfindung betrifft einen Trennkörper zum Trennen einer ersten von einer zweiten Phase einer Flüssigkeit in einem rohrförmigen Behälter. Gemeint sind insbesondere Trennkörper zum Trennen von Blutserum als erster Phase von Blutkuchen als zweiter Phase bei Blut als Flüssigkeit innerhalb eines Blutentnahmeröhrchens.

Im Stand der Technik sind Blutentnahmeröhrchen mit Trennkörpern grundsätzlich bekannt. Im Auslieferungszustand der Blutentnahmeröhrchen sind die Trennkörper in einer Ausgangsposition fixiert. Wenn Blut über einen Zulauf in das Blutentnahmeröhrchen hineinfließt, wird der Trennkörper von dem Blut umströmt oder durchströmt; in der Ausgangsposition stellt der Trennkörper jedenfalls keine Dichtung für das Blut innerhalb des Blutentnahmeröhrchens dar. Zur medizinischen Analyse ist es erforderlich, dass das Blut in zwei Bestandteile, nämlich Blutserum und Blutkuchen aufgetrennt wird. Zu diesem Zweck wird das Blutentnahmeröhrchen mit dem darin befindlichen Blut zentrifugiert. Der schwerere Blutkuchen setzt sich dann aufgrund der Zentrifugation in dem bodennahen Bereich des Blutentnahmeröhrchens ab, während das leichtere Blutserum auf dem Blutkuchen aufschwimmt. Unter Einwirkung der Zentrifugalkraft löst sich der Trennkörper aus seiner Ausgangsposition und wandert in eine Abdichtposition. Weil die Dichte des gesamten Trennkörpers in einem Wertebereich zwischen der Dichte des Blutserums und der Dichte des Blutkuchens liegt, positioniert sich der Trennkörper automatisch genau an der Phasengrenze zwischen Blutserum und Blutkuchen. Diese Position wird auch Abdichtposition genannt, weil der Trennkörper in dieser Position mit seinem Dichtrand umlaufend dichtend an der Innenseite des rohrförmigen Proberöhrchens anliegt und somit das Blutserum sauber von dem Blutkuchen trennt. Der Trennkörper hält diese Abdichtposition auch nach Ende des Zentrifugierens bei, so dass das Blutserum und der Blutkuchen für eine Laboruntersuchung getrennt zur Verfügung stehen. Trennkörper sind offenbart, z. B. in der internationalen Patentanmeldung WO 2010/132783 A1. Die dort beschriebenen Trennkörper weisen jeweils einen aus elastischem Material gefertigten Schwimmkörper mit einem in der Draufsicht kreisförmigen umlaufenden Dichtrand auf zum dichtenden Anliegen an der Innenseite eines rohrförmigen Probenbehälters in der Abdichtposition. An der Unterseite des Schwimmkörpers ist jeweils ein Ballastkörper befestigt. Die Dichte des Ballastkörpers ist jeweils größer als die Dichte des Schwimmkörpers und die Dichte des gesamten Trennkörpers liegt in einem Wertebereich zwischen der Dichte der ersten Phase und der Dichte der zweiten Phase der Flüssigkeit.

Aus dem Stand der Technik in Form der Druckschrift WO 2016/076911 A1 ist eine Trenneinheit für die Trennung einer Flüssigkeit in eine erste leichte und in eine zweite schwerere Phase unter Anwendung von Zentrifugalkraft bekannt, wobei die Flüssigkeit Blut sein kann. Ein rohrförmiger Behälter weist einen Trennkörper auf, wobei der Trennkörper im oberen Bereich einen Schwimmkörper und im unteren Bereich einen Ballastkörper aufweist. Der Trennkörper ist ausgebildet zum dichtenden Anliegen an der Innenseite des rohrförmigen Behälters. Die Dichte des Ballastkörpers ist dabei größer als die Dichte des Schwimmkörpers und die Dichte des Trennkörpers liegt zwischen der Dichte der ersten Phase und der Dichte der zweiten Phase der zu trennenden Flüssigkeit.

In der Druckschrift DE 699 31 584 T2 wird eine Vorrichtung zum Trennen einer Fluidprobe unter Zentrifugalkraft in eine Phase mit höherem spezifischen Gewicht und eine Phase mit niedrigerem spezifischen Gewicht beschrieben, wobei die Fluidprobe eine Blutprobe sein kann. Die Vorrichtung weist ein Separatorelement (Trennkörper) auf, das in einem zylindrischen Röhrchen angeordnet ist. Das Separatorelement weist einen Schwimmer im oberen Bereich und ein Ballastelement im unteren Bereich auf sowie einen Dichtungskörper zum dichtenden Anliegen an der Innenseite des Röhrchens. Die Dichte des Ballastelements ist dabei größer als die Dichte des Schwimmers und die Gesamtdichte des Separatorelements liegt zwischen der Dichte der ersten Phase und der Dichte der zweiten Phase der zu trennenden Flüssigkeit.

Die Druckschrift DE 600 23 823 T2 beinhaltet eine Vorrichtung zur Trennung einer flüssigen Probe (z. B. Blut) in eine erste Phase hoher Dichte und in eine Phase niedriger Dichte unter Einwirkung von Zentrifugalkraft. In einem Röhrchen mit zylindrischer Seitenwand ist ein Separator (Trennkörper) angeordnet, der im oberen Bereich einen Schwimmer und im unteren Bereich einen Ballastteil aufweist sowie ein Faltenbalg zum dichtenden Anliegen an der Innenseite des Röhrchens. Die Dichte des Ballastteils ist dabei größer als die Dichte des Schwimmers und die Gesamtdichte des Separators liegt zwischen den Dichten der ersten Phase und der zweiten Phase der zu trennenden Flüssigkeit. Der Schwimmer weist zwischen dem oberen und unteren Ende einen "schmalen Hals", d. h. eine lokale Verengung auf.

Die europäische Patentanmeldung EP 1 106 252 A2, die US-Patentanmeldung US 3 887 464 A und die internationale Patentanmeldung WO 2014/120678 A2 offenbarten jeweils die Merkmale gemäß dem Oberbegriff von Patentanspruch 1.

Der Erfindung liegt die Aufgabe zugrunde, einen alternativen Trennkörper vorzuschlagen.

Diese Aufgabe wird durch den Gegenstand des Patentanspruchs 1 gelöst. Demnach ist der erfindungsgemäße Trennkörper dadurch gekennzeichnet, dass der Schwimmkörper eine lokale Verengung aufweist und im Bereich der Verengung als Membran zum Abdichten des Schwimmkörpers im Bereich seiner Verengung gegenüber der Flüssigkeit ausgebildet ist.

Die beanspruchte Membran hat zwei Funktionen: Zum einen dichtet sie den Schwimmkörper im Bereich seiner Verengung gegenüber der Flüssigkeit ab; damit ist ein Durchfluss der Flüssigkeit durch den Schwimmkörper und auch durch den gesamten Trennkörper wirksam unterbunden. Zum anderen fungiert die Membran als Zugfeder insofern, als dass sie einer auf den Trennkörper einwirkenden Zugkraft, insbesondere Zentrifugalkraft, welche den Schwimmkörper und den Ballastkörper in der Flüssigkeit relativ zueinander auseinander zieht und den Trennkörper somit verschlankt, entgegenwirkt. Die Verschlankung ist notwendig, damit der Trennkörper, wenn er unter Einwirkung der Zentrifugalkraft von seiner Ausgangsposition in die Abdichtposition wandert, im Inneren des rohrförmigen Behälters nicht festklemmt. Bei einem Nachlassen oder Abschalten der Zugkraft bzw. der Zentrifugalkraft werden der Schwimmkörper und der Ballastkörper - auch aufgrund der Federkraft der Membran - wieder auf ihren ursprünglichen Abstand zurückgeführt, wodurch sich der Trennkörper in der Abdichtposition wieder verbreitert. Die Verbreiterung hat zur Folge, dass der Dichtrand des Schwimmkörpers mit ausreichendem Druck umlaufend dichtend an der Innenseite des rohrförmigen Behälters anliegt.

Die beschriebene erforderliche Federwirkung des Schwimmkörpers lässt sich mit der dünnen Membran leichter realisieren als mit einem voluminösen Vollkörper, weil sich die Membran leichter strecken lässt. Weiterhin ist es von Vorteil, dass die dünne Membran nur wenig Material benötigt. Allerdings fungiert die Membran kaum als Schwimm- bzw. Auftriebskörper. Diese Funktion übernimmt der Teil des Schwimmkörpers, welcher die Membran umgibt.

Sofern nichts anderes gesagt ist, wird der Trennkörper nachfolgend in einer Normalposition beschrieben. In dieser Normalposition ist der Ballastkörper unterhalb des Schwimmkörpers angeordnet. Der Schwerpunkt des Schwimmkörpers, der Schwerpunkt des Ballastkörpers und der Schwerpunkt des gesamten Trennkörpers liegen alle auf einer vertikalen Linie. Die nachfolgend verwendeten Begriffe wie vertikal, horizontal, unterhalb, Seitenansicht und Draufsicht etc. beziehen sich alle auf diese Normalposition. Die Abdichtposition entspricht der Normalposition bei senkrecht stehendem rohrförmigem Behälter.

Der Begriff lokale Verengung meint eine lokale Verjüngung oder Einschnürung. Der Schwimmkörper ist im Bereich seiner Verengung erfindungsgemäß auf die Membran reduziert.

Gemäß einem ersten Ausführungsbeispiel ist die Membran entweder elastisch und/oder - zumindest im unbelasteten Zustand - wellenförmig mit Wellenbergen und Wellentälern ausgebildet. Diese Ausbildung der Membran ermöglicht vorteilhafterweise die besagte Federwirkung der Membran.

Die Wellenberge und die Wellentäler der Membran können jeweils ringförmig ausgebildet sein; die Membran entfaltet dann ggf. Zugkräfte in radialer Richtung bezogen auf ihren Mittelpunkt. Alternativ können die Wellentäler und die Kämme der Wellenberge der Membran auch jeweils gradlinig und parallel zueinander verlaufend ausgebildet sein; die Membran entfaltet dann die besagten Zugkräfte, insbesondere in einer Richtung senkrecht zu den Wellenbergen und Wellentälern.

Aus fertigungstechnischer Sicht ist es von Vorteil, wenn die Membran aus demselben Material wie der Schwimmkörper, weiter vorzugsweise sogar einstückig mit dem Schwimmkörper ausgebildet ist. Dann ist der Schwimmkörper insbesondere als Spritzgussteil sehr leicht herstellbar.

Der Schwimmkörper kann kugelförmig oder topfförmig ausgebildet sein; wichtig ist jedoch, dass seine Außenkontur bei Seitenansicht - zumindest aus einer Blickrichtung betrachtet - von einer Kreisform abweicht. Dies ist deshalb wichtig, damit der Trennkörper in seiner Ausgangsposition, d. h. bei Auslieferung, in Umfangsrichtung nicht überall dichtend an der Innenseite des rohrförmigen Behälters anliegt, sondern dass die einströmende Flüssigkeit, insbesondere Blut, an dem Trennkörper vorbei in darunter befindliche Volumenbereiche des rohrförmigen Behälters fließen kann.

Die Oberflächen-Kontur des Schwimmkörpers ist insgesamt so gewählt, dass Flüssigkeit, insbesondere Blut, welches insbesondere den Bereich der Verengung bzw. der Membran benetzt, in den Behälter abfließen kann.

Der an dem Schwimmkörper ausgebildete umlaufende Dichtrand kann - in der Seitenansicht betrachtet - wellenförmig verlaufend ausgebildet sein; dies bietet den Vorteil, dass der Dichtrand zumindest abschnittsweise um die Verengung herumlaufend ausgebildet sein kann. Alternativ kann der Dichtrand auch - in einer Seitenansicht betrachtet - geradlinig bzw. horizontal verlaufend ausgebildet sein; er ist dann typischerweise oberhalb der Verengung angebracht.

Der Schwimmkörper kann an seiner dem Ballastkörper abgewandten Oberseite eine lokale Erhebung oder eine lokale Abflachung bzw. Sicke aufweisen. Beide alternativen Ausgestaltungen tragen dazu bei, dass die Kontur des Trennkörpers und insbesondere des Schwimmkörpers bei Seitenansicht von der reinen Kreisform abweicht. Dadurch wird, wie gesagt, ein Umströmen des Trennkörpers mit der Flüssigkeit insbesondere in seiner Ausgangsposition ermöglicht. So wird insbesondere vorteilhafterweise gewährleistet, dass Flüssigkeitsreste beispielsweise Blutreste abfließen, und sich nicht dort ansammeln.

Schließlich kann der Ballastkörper aus einem Material gefertigt sein, welches weniger elastisch ist als das Material des Schwimmkörpers oder das Material der Membran. Dies gilt deswegen, weil der besagte Dichtrand typischerweise an dem Schwimmkörper und nicht an dem Ballastkörper angebracht ist. Für die Funktion des Trennkörpers ist es wichtig, dass der Dichtrand unter Einwirkung der Zentrifugalkraft auf den Schwimmkörper - und damit auf den Dichtrand - elastisch verformbar ist, um ein Umströmen des Trennkörpers mit der Flüssigkeit innerhalb des rohrförmigen Behälters zu ermöglichen. Wenn der Dichtrand nicht auf dem Ballastkörper befestigt ist, wird diese Elastizität von dem Ballastkörper nicht gefordert; dieser kann deshalb weniger elastisch ausgebildet sein.

In allen Ausführungsformen des Trennkörpers kann die Oberfläche des Ballastkörpers jeweils eine vorbestimmte Haftreibungszahl aufweisen oder kann der Ballastkörper ein Haftelement aufweisen, welches an seiner Oberfläche die vorbestimmte Haftreibungszahl aufweist. Die Haftreibungszahl ist derart vorbestimmt, dass sich der Trennkörper nur dann aus seiner Ausgangsposition innerhalb des rohrförmigen Behälters löst, wenn auf ihn eine Kraft, insbesondere eine Zentrifugalkraft wirkt, welche größer als vorbestimmter Kraftschwellenwert ist.

Der Beschreibung sind sechs Figuren beigefügt, wobei
- Figur 1: den erfindungsgemäßen Trennkörper mit der Membran gemäß einem ersten Ausführungsbeispiel;
- Figur 2: den Trennkörper nach Figur 1 in einem rohrförmigen Behälter;
- Figuren 3a bis 3d: den erfindungsgemäßen Trennkörper mit der Membran gemäß einem zweiten Ausführungsbeispiel;
- Figur 4: den Trennkörper nach Figur 3 in dem rohrförmigen Behälter;
- Figuren 5a bis 5d: den erfindungsgemäßen Trennkörper mit der Membran gemäß einem dritten Ausführungsbeispiel und einer alternativen Gestaltung des Dichtrandes; und
- Figur 6: den Trennkörper nach Figur 5 in dem rohrförmigen Behälter
zeigt.

Die Erfindung wird nachfolgend unter Bezugnahme auf die genannten Figuren in Form von Ausführungsbeispielen detailliert beschrieben. In allen Figuren sind gleiche technische Elemente jeweils mit gleichen Bezugszeichen bezeichnet.

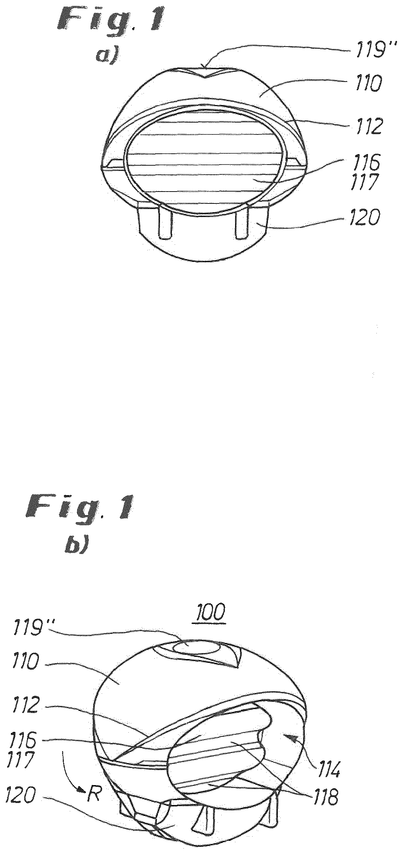

Figur 1 zeigt den erfindungsgemäßen Trennkörper 100 in kugelförmiger Ausgestaltung. Er besteht aus einem elastischen Schwimmkörper 110 mit einem in der Draufsicht kreisförmigen Dichtrand 112. Dem steht nicht entgegen, dass der Dichtrand 112 in der Seitenansicht nach Figur 1 wellenförmig ausgebildet ist. An der Unterseite des Schwimmkörpers 110 ist ein Ballastkörper 120 befestigt.

Die Dichte des Ballastkörpers 120 ist größer als die Dichte des Schwimmkörpers 110 und die Dichte des gesamten Trennkörpers 100 liegt in einem Wertebereich zwischen der Dichte der ersten Phase und der Dichte der zweiten Phase der Flüssigkeit. Die Dichte der zweiten Phase der Flüssigkeit ist größer als die Dichte der ersten Phase der Flüssigkeit. Für Blut als Flüssigkeit bedeutet dies, dass der Blutkuchen als zweite Phase eine größere Dichte aufweist als das Blutserum, welches der ersten Phase entspricht.

Gemäß Figur 1 ist der Schwimmkörper 110 lokal verengt. Im Bereich der Verengung 114 ist er als Membran 116 ausgebildet. Die Membran ist wellenförmig ausgebildet mit Wellenbergen 117 und Wellentälern 118. Unabhängig davon oder alternativ könnte die Membran 116 auch aus elastischem Material gebildet sein. Die Ausbildung der Membran in Wellenform und / oder aus elastischem Material ist erforderlich, um die oben beschriebene Federwirkung der Membran zu ermöglichen.

Bei dem in Figur 1 gezeigten Ausführungsbeispiel verlaufen die Wellentäler 118 und die Kämme der Wellenberge 117 der Membran 116 jeweils gradlinig und parallel zueinander (1. Variante). Ihre Federwirkung entfaltet die Membran deshalb bei Anordnung gemäß Figur 1 in vertikaler Richtung. An seiner dem Ballastkörper 120 abgewandten Oberseite weist der Schwimmkörper 110 eine lokale Abflachung oder Sicke 119" auf; diese ermöglicht ein dortiges lokales Umströmen des Trennkörpers mit der Flüssigkeit auch in seiner Ausgangsposition 210 innerhalb des rohrförmigen Behälters. Der in Umfangsrichtung R umlaufende Dichtrand 112 ist in Figur 1 - in Seitenansicht betrachtet - wellenförmig ausgebildet und ist zumindest abschnittsweise um den Rand der lokalen Verengung 114 herumgeführt.

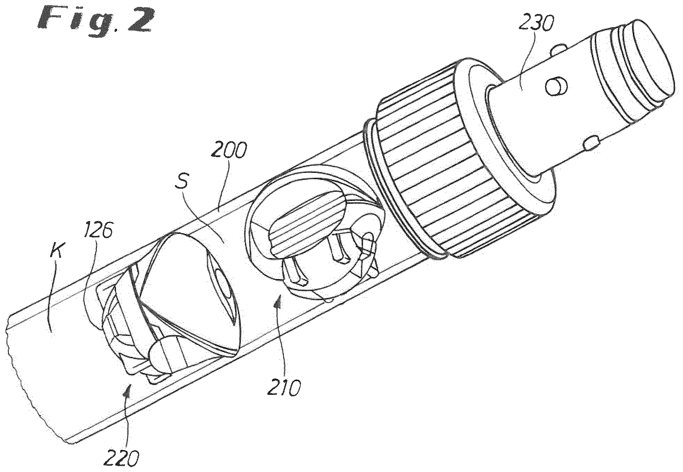

Figur 2 zeigt den Trennkörper 100 innerhalb des rohrförmigen Behälters 200. Bei dem rohrförmigen Behälter handelt es sich beispielsweise um ein Röhrchen zur Blutentnahme. Im Auslieferungszustand dieses rohrförmigen Behälters 200 ist der erfindungsgemäße Trennkörper 100 in einer Ausgangsposition 210 lösbar eingeklemmt. Er sitzt dann quer in dem Behälter. Aufgrund seiner nicht rein kreisförmigen Außenkontur bei Seitenansicht aus Richtung des Zulaufes 230 der Flüssigkeit wird der Trennkörper in dieser Ausgangsposition ggf. von der Flüssigkeit, insbesondere von Blut, umströmt, aber nicht durchströmt. Das in den Behälter einströmende Blut kann so problemlos auch in tiefere, unterhalb des Trennkörpers befindliche Volumenbereiche des Behälters abfließen.

Unter Einwirkung einer Kraft in Längsrichtung des rohrförmigen Behälters, insbesondere einer Zentrifugalkraft, löst sich der Trennkörper 100 aus seiner Ausgangsposition und wandert in die besagte Abdichtposition 220. Während des Zentrifugierens wird die erste Phase, beispielsweise Blutserum S von einer zweiten Phase, beispielsweise Blutkuchen K separiert und der Trennkörper 100 setzt sich aufgrund seiner Dichte genau auf die Grenze zwischen diesen beiden Phasen der Flüssigkeit. Die Bewegung des Trennkörpers von seiner Ausgangsposition in die Abdicht-position wird weiterhin dadurch erleichtert, dass sich der Trennkörper unter Einwirkung der Zentrifugalkraft ein wenig in vertikaler Richtung streckt und verschlankt, so dass dann während des Zentrifugierens und während der besagten Wanderung des Trennkörper dessen Dichtrand nicht umlaufend dicht an der Innenseite des rohrförmigen Behälters anliegt. Während seiner Wanderung von der Ausgangsposition in die Dichtposition dreht sich der Trennkörper um 90°. Erst nach Abschluss des Zentrifugierens, d. h. in der Dichtposition 220 entspannt sich der Trennkörper wieder. D. h., auch aufgrund der Zugkraft der Membran, nähern sich der Schwimmkörper und der Ballastkörper dann ein Stück weit wieder an, was zur Folge hat, dass sich der Trennkörper verbreitert, so dass der Dichtrand 112 umlaufend an der Innenseite des rohrförmigen Behälters 200 anliegt und in dieser Abdichtposition die erste Phase der Flüssigkeit von der zweiten Phase separiert.

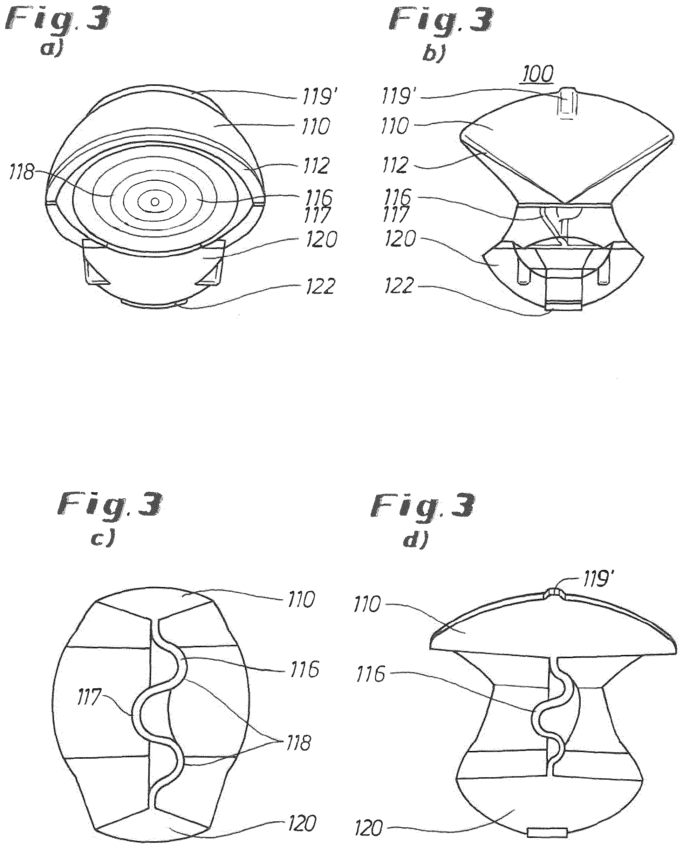

Figur 3 zeigt den erfindungsgemäßen Trennkörper in einer alternativen Ausführungsform. Konkret zeigen die Figuren 3a und 3b diese Ausführungsform jeweils in Seitenansicht, aber aus verschiedenen, um 90° versetzten Blickrichtungen. Die Figuren 3c und 3d zeigen den Trennkörper in der Seitenansicht gemäß Figur 3b, allerdings nicht in perspektivischer Ansicht, sondern in Längsschnitten verschiedener Tiefe. Der kugelförmige Trennkörper gemäß Figur 3 unterscheidet sich von dem kugelförmigen Trennkörper gemäß Figur 1 zum einen in der Ausbildung der Membran 116 und zum anderen in der Gestaltung der Oberseite des Schwimmkörpers. Die Wellenberge 117 und die Wellentäler 118 der Membran sind hier nicht gradlinig, sondern ringförmig und im Wesentlichen koaxial zueinander ausgebildet (2. Variante). Dies bietet den Vorteil, dass auch die Federwirkung dieser Membran nicht eindimensional, sondern zweidimensional, nämlich radial zum Zentrum der Membran ist Anders als der Trennkörper gemäß Figur 1 zeigt der Trennkörper 100 gemäß Figur 3 auf der Oberfläche des Schwimmkörpers keine Abflachung, sondern eine lokale Erhebung 119'. Mit dieser lokalen Erhebung liegt der Trennkörper 100 in der Ausgangsposition 210 an der Innenseite des rohrförmigen Behälters 200 an. Aufgrund dieser lokalen Erhebung 119' weicht die äußere Kontur des Trennkörpers und insbesondere des Schwimmkörpers 110 von der einer reinen Kreisform ab; dies trägt vorteilhafterweise dazu bei, dass der Trennkörper auch in diesem Bereich mit der Oberfläche des Schwimmkörpers nicht dichtend an der Innenseite des rohrförmigen Behälters anliegt, sondern stattdessen auch dort von der Flüssigkeit umströmt werden kann.

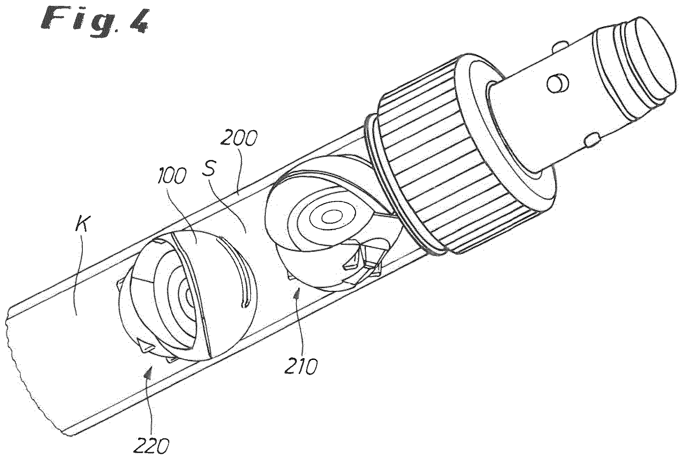

Figur 4 zeigt den rohrförmigen Behälter 200 mit dem darin befindlichen Trennkörper 100 gemäß Figur 3. In der Ausgangsposition 210 liegt der Trennkörper 100, wie gesagt, insbesondere mit der lokalen Erhebung 119' an der Innenseite des Behälters 200 an. Ansonsten wird für die Figur 4 auf die Beschreibung der Figur 2 verwiesen, welche für die Figur 4 analog gilt.

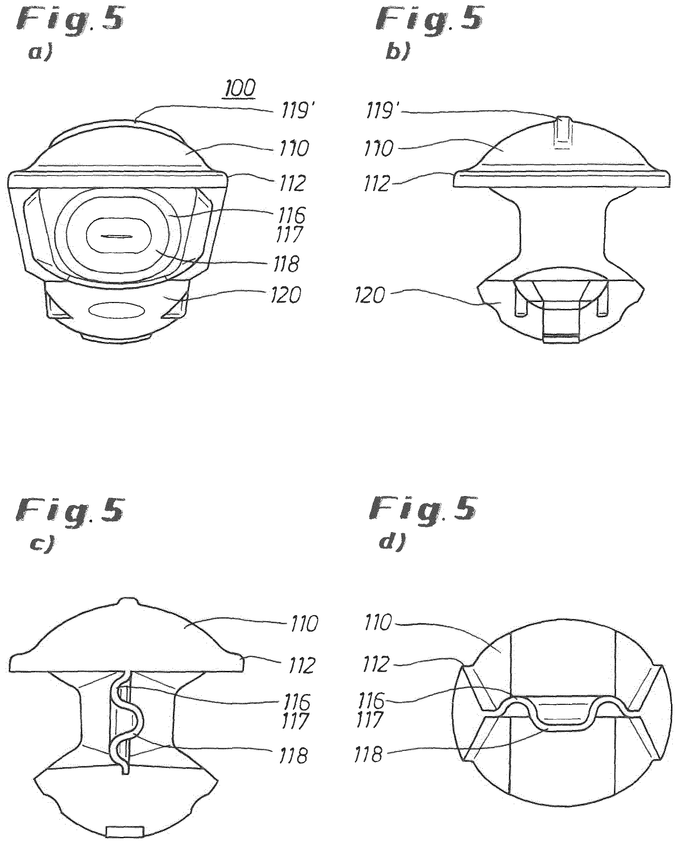

Figur 5 zeigt den erfindungsgemäßen Trennkörper 100 in einer weiteren Ausführungsform. Anders als in den Figuren 1 und 3 ist der Schwimmkörper 110 hier topfförmig ausgebildet. Die Figuren 5a und 5b zeigen den topfförmigen Trennkörper jeweils in unterschiedlichen Seitenansichten. Die Figuren 5c und 5d zeigen den topfförmigen Trennkörper jeweils in verschiedenen Schnittdarstellungen. Die Wellenberge 117 und die Wellentäler 118 der Membran 116 sind zwar auch hier ringförmig, allerdings oval ausgebildet (3. Variante). Weiterhin ist der Dichtrand 112, wie in den Seitenansichten gemäß Figur 5a und 5b gezeigt, gradlinig horizontal verlaufend ausgebildet.

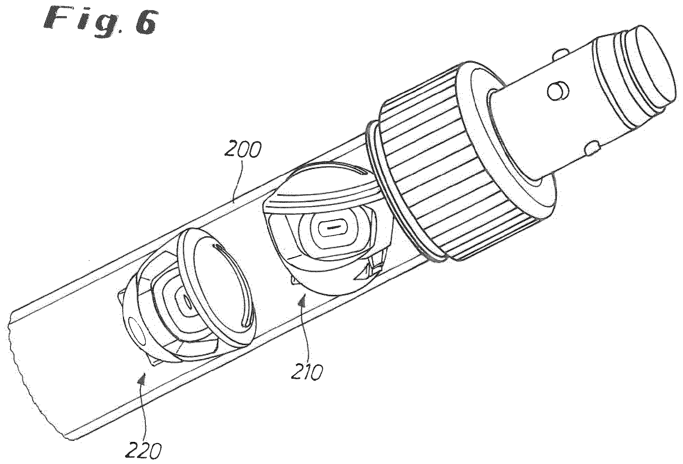

Für die Figur 6 gilt die Beschreibung der Figur 4 in Verbindung mit der Beschreibung der Figur 2 analog.

Die Offenbarung der Beschreibung ist nicht auf die beschriebenen Ausführungsbeispiele beschränkt. Vielmehr können insbesondere die beschriebenen alternativen Ausgestaltungen der Membran, des Dichtrandes und des Schwimmkörpers mit Abflachungen oder Erhebungen beliebig miteinander kombiniert werden.

### Bezugszeichenliste

- 100: Trennkörper
- 110: Schwimmkörper
- 112: Dichtrand
- 114: Verengung
- 116: Membran
- 117: Wellenberg der Membran
- 118: Wellental der Membran
- 119': lokale Erhebung
- 119": lokale Abflachung
- 120: Ballastkörper
- 200: rohrförmiger Behälter
- 210: Ausgangsituation bzw. Auslieferungszustand
- 220: Abdichtposition
- 230: Zulauf

- K: Blutkuchen
- R: Umfangsrichtung
- S: Serum

## Patentansprüche

1. Trennkörper (100) zum Trennen einer ersten von einer zweiten Phase einer Flüssigkeit, insbesondere zum Trennen von Blutserum (S) von Blutkuchen (K) bei Blut als Flüssigkeit, unter Zentrifugalkraft in einem rohrförmigen Behälter (200); aufweisend:
einen aus elastischem Material gefertigten Schwimmkörper (110) mit einem in der Draufsicht umlaufenden Dichtrand (112) zum dichtenden Anliegen an der Innenseite des rohrförmigen Behälters (200) in einer Abdichtposition (220); und
mindestens einen an der Unterseite des Schwimmkörpers (110) befestigten Ballastkörper (120);
wobei die Dichte des Ballastkörpers (120) größer ist als die Dichte des Schwimmkörpers (110) und;
wobei die Dichte des gesamten Trennkörpers (100) in einem Wertebereich zwischen der Dichte der ersten Phase und der Dichte der zweiten Phase der Flüssigkeit liegt;
**dadurch gekennzeichnet,**
**dass** der Schwimmkörper (110) eine lokale Verengung (114) aufweist und im Bereich der Verengung als Membran (116) zum Abdichten des Schwimmkörpers im Bereich seiner Verengung gegenüber der Flüssigkeit ausgebildet ist.

2. Trennkörper (100) nach Anspruch 1,
**dadurch gekennzeichnet, dass**
die Membran (116) elastisch und/oder - zumindest in unbelastetem Zustand - wellenförmig mit Wellenbergen (117) und Wellentälern (118) ausgebildet ist.

3. Trennkörper (100) nach Anspruch 2,
**dadurch gekennzeichnet, dass**
die Wellenberge (117) und Wellentäler (118) der Membran (116) ringförmig ausgebildet sind.

4. Trennkörper (100) nach Anspruch 2,
**dadurch gekennzeichnet, dass**
die Wellentäler (118) und die Kämme der Wellenberge (117) der Membran (116) jeweils gradlinig und parallel zu einander verlaufend ausgebildet sind.

5. Trennkörper (100) nach einem der vorangegangenen Ansprüche,
**dadurch gekennzeichnet, dass**
die Membran (116) aus demselben Material wie der Schwimmkörper (110) hergestellt ist.

6. Trennkörper (100) nach Anspruch 5,
**dadurch gekennzeichnet, dass**
die Membran (116) einstückig mit dem Schwimmkörper (110) ausgebildet ist.

7. Trennkörper (100) nach einem der vorangegangenen Ansprüche,
**dadurch gekennzeichnet,**
**dass** der Schwimmkörper (110) kugelförmig oder topfförmig ausgebildet ist.

8. Trennkörper (100) nach Anspruch 7,
**dadurch gekennzeichnet, dass**
der Schwimmkörper (100) an seiner dem Ballastkörper (120) abgewandten Oberseite eine lokale Erhebung (119') oder eine lokale Abflachung (119") aufweist.

9. Trennkörper (100) nach einem der vorangegangenen Ansprüche,
**dadurch gekennzeichnet, dass**
der Dichtrand (112) - in der Seitenansicht betrachtet - in Umfangsrichtung (R) wellenförmig verlaufend und zumindest abschnittsweise um den Rand der Verengung (114) herumlaufend ausgebildet ist.

10. Trennkörper (100) nach einem der Ansprüche 1 bis 8,
**dadurch gekennzeichnet, dass**
der umlaufende Dichtrand (112) - in der Seitenansicht betrachtet - gerade und vorzugsweise horizontal verlaufend ausgebildet ist.

11. Trennkörper (100) nach einem der vorangegangenen Ansprüche,
**dadurch gekennzeichnet, dass**
der Ballastkörper (120) aus einem Material gefertigt ist, welches weniger elastisch ist als das Material des Schwimmkörpers (110) oder das Material der Membran (116).

## Claims

1. Separating body (100) for separating a first from a second phase of a liquid, in particular for separating blood serum (S) from blood clots (K) in blood as a liquid, by the application of centrifugal force in a tubular container (200); comprising:
a float (110) made of elastic material having a sealing edge (112) extending around the circumference in the plan view for sealing engagement with the inside of the tubular container (200) in a sealing position (220); and
at least one ballast body (120) attached to the underside of the float (110);
wherein the density of the ballast body (120) is greater than the density of the float (110) and;
wherein the density of the entire separating body (100) is in a range of values between the density of the first phase and the density of the second phase of the liquid;
**characterized in that**
the float (110) has a local constriction (114), and is constructed as a membrane (116) in the region of the constriction in order to seal the float off from the liquid in the region of its constriction.

2. Separating body (100) according to Claim 1,
**characterized in that**
the membrane (116) is elastically and/or - at least in unloaded state - has a wave-like form with wave crests (117) and wave troughs (118).

3. Separating body (100) according to Claim 2,
**characterized in that**
the wave crests (117) and wave troughs (118) of the membrane (116) are formed in rings.

4. Separating body (100) according to Claim 2,
**characterized in that**
the wave troughs (118) and the peaks of the wave crests (117) of the membrane (116) are each designed so that they extend in a straight line and parallel to each other.

5. Separating body (100) according to any one of the preceding claims,
**characterized in that**
the membrane (116) is made from the same material as the float (110).

6. Separating body (100) according to Claim 5,
**characterized in that**
the membrane (116) is designed as a single part with the float (110).

7. Separating body (100) according to any one of the preceding claims,
**characterized in that**
the float (110) is spherical or pot-shaped.

8. Separating body (100) according to Claim 7,
**characterized in that**
the surface of the float (100) farthest from the ballast body (120) has a local elevated region (119') or a local flattened region (119").

9. Separating body (100) according to any one of the preceding claims,
**characterized in that**
the sealing edge (112) is constructed such that - from the perspective of the side view - it has a wavelike form in the circumferential direction (R) and it extends at least partially around the perimeter of the constriction (114).

10. Separating body (100) according to any one of Claims 1 to 8,
**characterized in that**
the circumferential sealing edge (112) - from the perspective of the side view - is constructed to extend straight and preferably horizontally.

11. Separating body (100) according to any one of the preceding claims,
**characterized in that**
the ballast body (120) is made from a material which is less elastic than the material of the float (110) or the material of the membrane (116).

## Revendications

1. Élément séparateur (100), destiné à séparer une première et une deuxième phases d'un liquide, destiné à séparer notamment du sérum sanguin (S) d'une masse de sang (K), le liquide étant du sang, sous l'effet d'une forme centrifuge, dans un récipient (200) tubulaire ; comportant :
un élément flottant (110) fabriqué en une matière élastique, doté d'un bord d'étanchéité (112) de forme périphérique, vu en élévation, destiné à s'appuyer en assurant l'étanchéité sur la face interne du récipient (200) tubulaire dans une position d'étanchéité (220) ; et
au moins un élément de lestage (120) fixé sur la face inférieure de l'élément flottant (110) ;
la densité de l'élément de lestage (120) étant supérieure à la densité de l'élément flottant (110) et ;
la densité de l'ensemble de l'élément séparateur (100) se situant dans un ordre de valeurs compris entre la densité de la première phase et la densité de la deuxième phase du liquide ;
**caractérisé en ce que**
l'élément flottant (110) comporte un rétrécissement (114) local et dans la zone du rétrécissement, est conçu sous la forme d'une membrane (116), destinée à assurer l'étanchéité de l'élément flottant par rapport au liquide dans la zone de son rétrécissement.

2. Élément séparateur (100) selon la revendication 1,
**caractérisé en ce que**
la membrane (116) est conçue en étant élastique et/ou (au moins à l'état non contraint) en forme d'ondes, avec des crêtes d'ondes (117) et des creux d'ondes (118) .

3. Élément séparateur (100) selon la revendication 2,
**caractérisé en ce que**
les crêtes d'ondes (117) et les creux d'ondes (118) de la membrane (116) sont conçus de forme annulaire.

4. Élément séparateur (100) selon la revendication 2,
**caractérisé en ce que**
les creux d'ondes (118) et les arêtes des crêtes d'ondes (117) de la membrane (116) sont conçu(e)s en étant respectivement rectilignes et en s'écoulant à la parallèle les uns(e)s des autres.

5. Élément séparateur (100) selon l'une quelconque des revendications précédentes,
**caractérisé en ce que**
la membrane (116) est fabriquée dans la même matière que l'élément flottant (110).

6. Élément séparateur (100) selon la revendication 5,
**caractérisé en ce que**
la membrane (116) est conçue d'un seul tenant avec l'élément flottant (110).

7. Élément séparateur (100) selon l'une quelconque des revendications précédentes,
**caractérisé en ce que**
l'élément flottant (110) est conçu de forme sphérique ou en forme de cuvette.

8. Élément séparateur (100) selon la revendication 7,
**caractérisé en ce que**
sur sa face supérieure opposée à l'élément de lestage (120), l'élément flottant (100) comporte une élévation (119') locale ou un méplat (119") local.

9. Élément séparateur (100) selon l'une quelconque des revendications précédentes,
**caractérisé en ce que**
(considéré en vue latérale), le bord d'étanchéité (112) est conçu en s'écoulant en forme d'ondes dans la direction périphérique (R) et est conçu au moins par endroits de sorte à entourer le bord du rétrécissement (114) .

10. Élément séparateur (100) selon l'une quelconque des revendications 1 à 8,
**caractérisé en ce que**
(considéré en vue latérale), le bord d'étanchéité (112) est conçu en étant droit et en s'écoulant de préférence à l'horizontale.

11. Élément séparateur (100) selon l'une quelconque des revendications précédentes,
**caractérisé en ce que**
l'élément de lestage (120) est fabriqué en une matière qui est moins élastique que la matière de l'élément flottant (110) ou la matière de la membrane (116).
